# EUROPEAN PATENT APPLICATION

(11) **EP 4 813 296 A1**
(43) Date of publication of application: **30.09.2026**
(21) Application number: 25465520.2
(22) Date of filing: 27.03.2025
(51) Int. Cl.: A61B 5/363, A61B 5/00, G16H 50/20

(54) **VENTRICULAR TACHYCARDIA EXIT SITE IDENTIFICATION METHOD AND APPARATUS**

(71) Applicant: Varian Medical Systems Inc, Palo Alto CA 94304 (US)
(72) Inventor: Meister, Felix, 91054 Erlangen (DE); Passerini, Tiziano, Plainsboro, 08536 (US); Ciusdel, Costin Florian, 105100 Azuga (RO)
(74) Representative: Mathisen & Macara LLP

(57) **Abstract**

To non-invasively identify a ventricular tachycardia exit site in a heart of a particular patient (107) using electrocardiogram signals and medical imagery for the particular patient, a control circuit (101) can be configured to access (205) a neural network (104) that has been trained (202) using a plurality of synthetic data examples (203) and to input information regarding the electrocardiogram signals and the aforementioned medical imagery for the particular patient to the neural network. The control circuit can then output from the neural network information that identifies a predicted ventricular tachycardia exit site in the heart of the particular patient.

## Description

### TECHNICAL FIELD

These teachings relate generally to treating ventricular tachycardia and more particularly to identifying ventricular tachycardia exit sites in a patient's heart.

### BACKGROUND

Ventricular tachycardia is a cardiac condition that can be fatal if left untreated. There are several treatment options including antiarrhythmic drugs, invasive ablation procedures, and non-invasive radiation therapy.

Treatment options like catheter-based ablation and radiation therapy can play a role in the management of patients who experience ventricular tachycardia post myocardial infarction. The latter serve to locally alter cardiac tissue to disable responsible wave re-entry circuits. Treatment success can be highly dependent on how accurately the responsible pathways are identified. In particular, it can be useful to localize the exit site of the ventricular tachycardia circuit in the heart.

One of the best existing ways to assess the functional, electrophysiological behavior of cardiac tissue is electroanatomic mapping (EAM). Unfortunately, EAM poses its own set of challenges. For example, the measurement process itself is invasive, and high-resolution maps are difficult and time-consuming to obtain.

### BRIEF DESCRIPTION OF DRAWINGS

The above needs and others are at least partially met through provision of the ventricular tachycardia exit site method and apparatus described in the following detailed description, particularly when studied in conjunction with the drawings, wherein:
FIG. 1 comprises a block diagram as configured in accordance with various embodiments of these teachings;
FIG. 2 comprises a flow diagram as configured in accordance with various embodiments of these teachings; and
FIG. 3 comprises a schematic flow diagram as configured in accordance with various embodiments of these teachings.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

### DETAILED DESCRIPTION

Generally speaking, these various embodiments provide a way to non-invasively identify a ventricular tachycardia exit site in a heart of a particular patient using electrocardiogram signals and medical imagery for the particular patient. By one approach, a control circuit can be configured to access a neural network that has been trained using a plurality of synthetic data examples and to input information regarding the electrocardiogram signals and the aforementioned medical imagery for the particular patient to the neural network. The control circuit can then output from the neural network information that identifies a predicted ventricular tachycardia exit site in the heart of the particular patient.

By one approach, the aforementioned information regarding the electrocardiogram signals can include information regarding physical placement of electrocardiogram electrodes on the particular patient. In lieu of the foregoing or in combination therewith, by one approach the electrocardiogram signals can comprise electrocardiogram signals from no more than twelve electrocardiogram leads (though signals from more than twelve electrocardiogram leads can be accommodated if desired).

By one approach, the aforementioned medical imagery can comprise three-dimensional imagery of at least part of the heart. For example, the medical imagery can comprise at least one of at least one computed tomography image of at least part of the heart and/or at least one magnetic resonance imaging image of at least part of the heart.

These teachings are flexible in practice. As one example in these regards, the aforementioned predicted ventricular tachycardia exit site in the heart of the particular patient can comprise one or more of a particular region comprising a predefined subdivision of the heart, a heat map that indicates at least one likelihood of a ventricular tachycardia exit site, and/or three-dimensional location coordinates.

These teachings will also accommodate training the aforementioned neural network using synthetic data examples. By one approach, at least some of the synthetic data examples can include electrogram information. By one approach, the aforementioned inputting of the information regarding the electrocardiogram signals and the medical imagery for the particular patient to the neural network can further include inputting information regarding electrogram information for the particular patient to the neural network.

By one approach, at least some of the synthetic data examples can include examples that each comprise information regarding electrocardiogram signals for a representative patient, medical imagery for the representative patient, and at least one labeled ventricular tachycardia exit site. By one approach, at least some of those synthetic data examples can be generated using statistical shape modeling to increase variability of at least one of cardiac and torso anatomy instantiations, medical imagery instantiations, and/or labeled ventricular tachycardia exit site instantiations.

If desired, in addition to training the neural network using synthetic data examples, the training corpus can be expanded to include at least one non-synthetic data example.

By one approach, these teachings can comprise a computer program that itself comprises instructions that, when the computer program is executed by a computer, causes the computer to carry out accessing a neural network that has been trained using a plurality of synthetic data examples and inputting information regarding the electrocardiogram signals and the aforementioned medical imagery for the particular patient to the neural network, followed by outputting from the neural network information that identifies a predicted ventricular tachycardia exit site in the heart of the particular patient.

So configured, these teachings provide for identifying ventricular tachycardia exit sites in a non-invasive manner but with a degree of precision that can rival electroanatomic mapping.

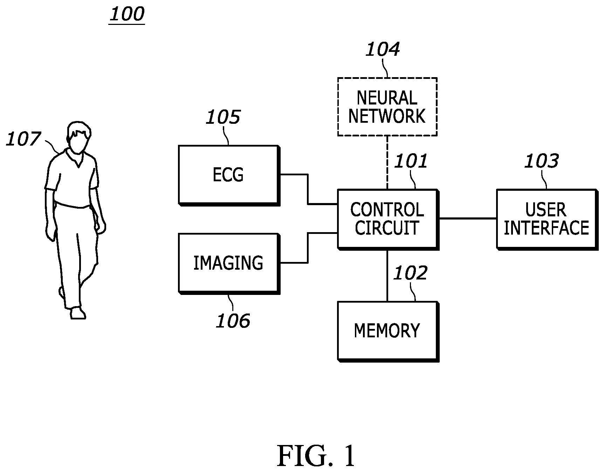

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

This memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both non-volatile memory (such as read-only memory (ROM) as well as volatile memory (such as a dynamic random access memory (DRAM).)

The control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

The control circuit 101 also operably couples to, or itself is configured as, at least in part, a neural network 104.

In this illustrative example, the control circuit 101 can operably couple to an electrocardiogram platform 105 and one or more medical imaging systems 106.

The electrocardiogram platform 105 serves to deliver an electrocardiogram. An electrocardiogram platform is a non-invasive diagnostic tool that serves to record the electrical activity of the heart over a period of time. By placing electrodes on the patient's 107 skin at specific points on the body, the platform captures the timing and strength of electrical impulses that travel through the heart with each beat. The resulting graph, with its characteristic P, QRS, and T waves, provides information about such things as the heart's rhythm, the presence of any irregularities, and the condition of the heart muscle.

The aforementioned electrodes are connected to an electrocardiogram recording device. The device reports electrical signals in multiple leads, each lead representing electrical activity between a positive and a negative pole. There are twelve standard leads in a typical electrocardiogram platform: three bipolar limb leads (I, II, III), three augmented unipolar limb leads (aVR, aVL, aVF), and six unipolar chest leads (V1-V6). Each lead provides unique information about the heart's electrical axis, rhythm, and any potential abnormalities such as arrhythmias, ischemia, or infarction.

In this illustrative example, the electrocardiogram platform 105 provides electrocardiogram information for a corresponding patient 107 to the aforementioned control circuit 101.

The aforementioned imaging system 106 captures and provides medical imagery for a particular patient 107. Examples of such systems include computed tomography systems and magnetic resonance imaging systems. Generally speaking, this description presumes, for the sake of an illustrative example, that the medical imagery captured by the imaging system 106 comprises, at least in part, three-dimensional imagery of at least part of the patient's heart. These teachings will accommodate, however, medical imagery that captures the entirety of the patient's heart as well as part or all of the patient's torso (as well as some or all of any electrocardiogram electrodes that are disposed on the patient's body).

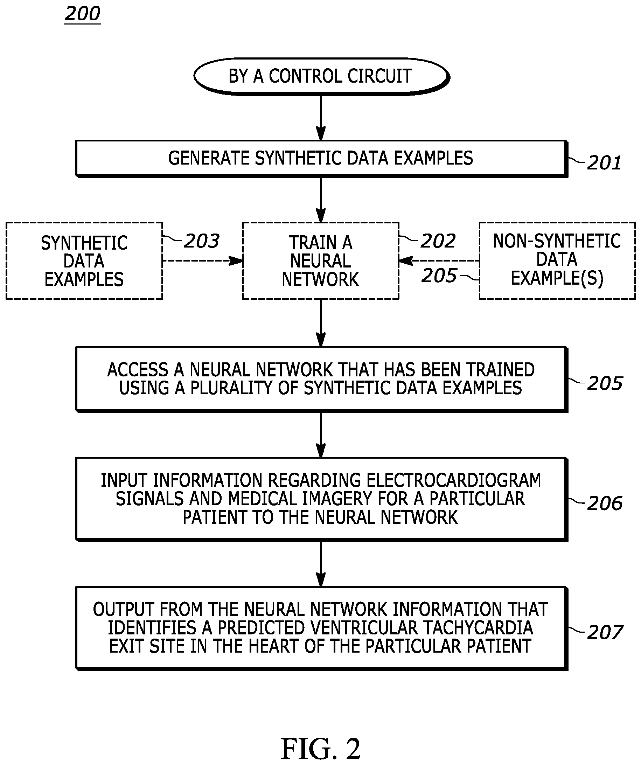

Referring now to FIG. 2, a process 200 that can be carried out, for example, in conjunction with the above-described application setting (and more particularly via the aforementioned control circuit 101 and neural network 104) will be described. Generally speaking, this process 200 serves to non-invasively identify a ventricular tachycardia exit site in a patient's heart using electrocardiogram signals and medical imagery for that particular patient.

At optional block 201, this process 200 can generate one or more synthetic data examples. These synthetic data examples can be used later as part (or all) of a training corpus for the above-mentioned neural network 104.

By one approach, at least some of the synthetic data examples can be generated using statistical shape modeling to increase variability as regards one or more represented features. Examples in these regards include, but are not limited to, variations with respect to information regarding electrocardiogram signals, cardiac and torso anatomy instantiations, medical imagery instantiations, and labeled ventricular tachycardia exit site instantiations. Statistical shape modeling is a mathematical method used to analyze and interpret the variability of shapes within a given class of objects. This approach can involve capturing the geometric information of a collection of similar shapes, such as human organs, and creating a statistical model that can describe the typical shape (mean) and the principal modes of variation within the dataset.

Optional block 202 provides for training the neural network 104. The training material includes synthetic data examples 203 that may, or may not, have been at least partially generated at block 201. The synthetic data examples 103 can include examples that each comprise information regarding electrocardiogram signals for a representative patient (where the representative patient may correspond to a real flesh-and-blood patient or a virtual patient), medical imagery for the representative patient, and at least one labeled ventricular tachycardia exit site. The synthetic data examples 103 can also include, if desired, physical placement instantiations of electrocardiogram electrodes on the body of the representative patient.

If desired, these teachings will also accommodate using at least one or more non-synthetic data examples 204 in the training corpus as well.

At block 205, the control circuit 101 accesses a trained neural network 104 that was trained, at least in part, using a plurality of synthetic data examples (for example, described above). At block 206, the control circuit 101 inputs information regarding electrocardiogram signals and medical imagery for a particular patient 107 to the neural network 104. This input information will typically be both real and relatively current/recent. In many cases both information modalities will have been captured the same day as the information is input to the neural network 104.

Generally speaking, the information regarding electrocardiogram signals for the particular patient 107 can include information regarding physical placement of electrocardiogram electrodes on the particular patient 107, and in many application settings the electrocardiogram signals comprise electrocardiogram signals from no more than twelve electrocardiogram leads. In at least some application settings it can be beneficial if the electrocardiogram signals are from exactly twelve electrocardiogram leads. If desired, these teachings will also readily accommodate application settings where more than twelve electrocardiogram leads are utilized.

Also generally speaking, the aforementioned information regarding the patient's medical imagery will comprise, at least in part, three-dimensional imagery of at least part of the patient's heart and may comprise at least one of a computed tomography image of at least part of the heart and at least one magnetic resonance imaging image of at least part of the heart.

At block 207, the neural network 104 outputs information that identifies a predicted ventricular tachycardia exit site in the heart of the particular patient 107. This identifying information can assume any of a variety of forms as desired. By one approach, the identifying information comprises an image of a particular region that comprises a predefined subdivision of the heart. By another approach, in lieu of the foregoing or in combination therewith, the identifying information can comprise a heat map that indicates at least one likelihood of a ventricular tachycardia exit site. (A heat map is a data visualization tool that typically uses color gradients to represent various values of a metric of interest. Varying colors on the heat map correspond to the magnitude of the metric being observed, with certain colors indicating, for example, an increased likelihood of a particular area in the heart being a ventricular tachycardia exit site.) By yet another approach, and again in lieu of the foregoing or in combination with the above, the identifying information comprises three-dimensional location coordinates that specify a particular three-dimensional location on the patient's heart.

Further details that comport with these teachings will now be presented. It will be understood that the specific details of these examples are intended to serve an illustrative purpose and are not intended to suggest any particular limitations with respect to these teachings.

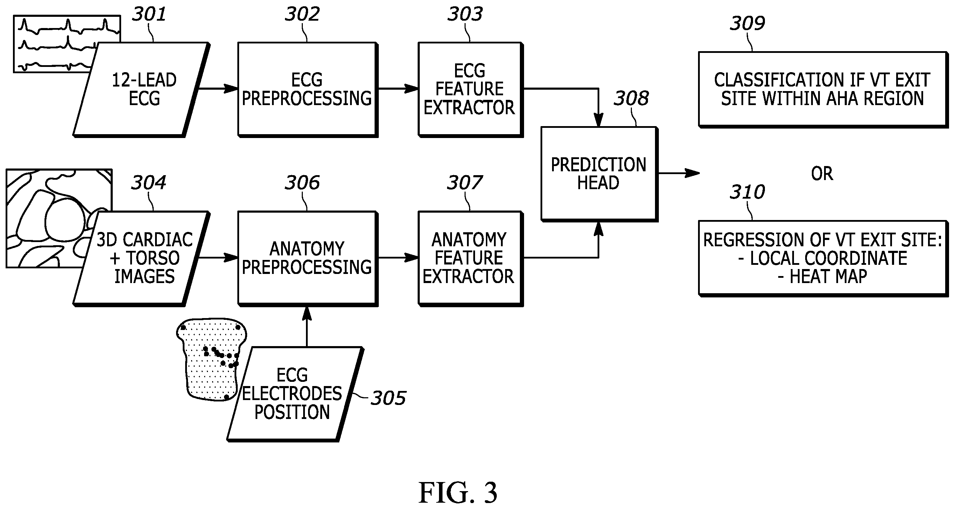

FIG. 3 presents one approach to accurately and non-invasively estimating ventricular tachycardia exit sites using 12-lead electrocardiogram traces and anatomy information that is derived from medical images. Deep learning serves in this example to fuse the information of the 12-lead electrocardiogram traces and the patient's anatomy.

12-lead electrocardiogram traces 301 for a particular patient (in the form of print-outs and/or digitized signals) are preprocessed at block 302 to remove, normalize, and/or mitigate baseline wandering, powerline inferences, respiratory effects, and so forth.

At block 303 the preprocessed electrocardiogram information is processed to extract desired features. By one approach, the aforementioned features can comprise discriminative features as specified by a particular user/clinic. By one approach, some or all of these features can be defined by the literature and/or the expertise of a cardiologist and they are extracted using traditional electrocardiogram processing algorithms, such as peak detection or wave delineation algorithms. Exemplary features could be the amplitudes of each of the 12 leads, the QRS duration, QRS morphology, electrical axis, among others (where the "QRS" complex will be understood to refer to the series of waves seen on an electrocardiogram tracing that represent the depolarization of the ventricles of the heart). By another approach, particular features could be directly learned from raw signals using deep learning methods, such as temporal convolutional networks or recurrent networks.

Three-dimensional cardiac and torso images 304 for the same patient and information 305 about the placement of the electrocardiogram electrodes on the patient are preprocessed at block 306. This preprocessing can comprise applying automatic segmentation algorithms to identify particular parts of the heart or the patient's body. By one approach, the resulting segmentation mask could denote the biventricular cardiac chambers, the skin surface of the torso, and the electrocardiogram electrodes if present in the three-dimensional medical images 304. By another approach, where the electrocardiogram electrodes are not shown in the three-dimensional medical images 304, but a capture of an RGB-D camera (which is a camera that typically provides both color and depth information) is available, the precise electrocardiogram electrode positions can be inferred by solving a registration problem between the depth map and the segmented torso surface. A segmentation algorithm can then extract the electrocardiogram electrodes from the RGB-D image and use the registration matrix to project the electrode locations onto the torso surface. By yet another approach, the torso and electrocardiogram electrodes are given by an atlas model with standardized electrocardiogram placement that is registered to the segmented cardiac anatomy, which may help to apply the algorithm in the absence of three-dimensional medical images that show the full torso and the electrocardiogram electrode positions. And by yet another approach, the segmentation masks could be further preprocessed to extract meshes. The heart could be represented by a dense mesh, such as a tetrahedral mesh. The torso could be represented by a triangulated surface representation and the electrocardiogram electrodes could be integrated via annotated vertices.

At block 307 the preprocessed image content has one or more features extracted therefrom. By one approach, when the anatomy information includes segmentation masks (such as, for example, grid-structured data), the features can be extracted using a convolutional neural network. By another approach, when the data is given as dense or surface meshes, the features can be extracted using graph convolutional neural networks.

The extracted features from both block 303 and block 307 are input to a prediction head 308 such as the above-described neural network. By one approach, the neural network comprises a fully-connected neural network (also sometimes known as a multilayer perceptron neural network). By one approach, a fusion of information can be realized by concatenating the aforementioned feature vectors before inputting that content to the neural network. By another approach, a fusion of information can be realized by conditioning the neural network with the electrocardiogram features (for example, by applying a feature-wise linear modulation). The neural network may then infer ventricular tachycardia exit sites. By one approach, the latter may be realized as a classification task, in particular, by classifying in which region of a predefined subdivision of the heart a given ventricular tachycardia exit site originates. By another approach, the neural network may be adapted to infer a heat map that reflects the likelihood of ventricular tachycardia exit site origins. And in yet another embodiment, the neural network may output a precise location represented, for example, as three-dimensional coordinates in a local coordinate system.

By one approach, in accordance with the particular training utilized, the prediction head 308 outputs classification information 309 as to whether a ventricular tachycardia exit site is within an American Heart Association ventricle segment of the patient's heart. By another approach, the prediction head 308 outputs location information 310 regarding a ventricular tachycardia exit site such as local coordinates information and/or a corresponding heat map.

These teachings are highly flexible in practice and will accommodate various modifications and/or supplemental features.

As one example in these regards, the aforementioned training may comprise joint training. By one approach, for example, the neural network may be trained on a large dataset of actual 12-lead electrocardiogram traces (print-outs or digitized signals), three-dimensional medical images, torso imaging (three-dimensional medical images or RGB-D camera images), and ground truth labels derived from electrophysiological data (where ground truth labels refer to clinical information where an expert labeled the exit sites), such as high-resolution electroanatomical maps.

As another example in these regards, and when there exists a lack of a large amount of real-world data, a cardiac computation model may be used to generate a large data set of noise-free ground truth. To this end, statistical shape modeling may be used to increase the variability of the cardiac and the torso anatomy. Scar and border zone distributions can be added by either leveraging available three-dimensional late gadolinium enhancement magnetic resonance imaging images or by applying corresponding rule-based algorithms.

To reflect imperfections in electrocardiogram electrode placement, the standard virtual electrocardiogram electrode placement on a torso model can be augmented by either shifting the placement pattern or by randomly shifting individual electrodes. Given the anatomical data, various electrophysiological cardiac activation sequences and associated virtual electrocardiograms can be generated by randomizing the inhomogeneous tissue conductivities and the pacing locations, among others. In addition, to enable robust feature extraction from print-outs of 12-lead electrocardiogram traces, these teachings will accommodate rendering virtual electrocardiograms using, for example, pre-defined templates that capture the variability of clinical print-outs.

And as yet another example in these regards, these teachings will accommodate incorporating electrogram information (which may be available in some instances, for example, from a device that is implanted in the patient) to increase accuracy of the predictions provided by the neural network. As one example in these regards, electrogram information can be provided to the neural network as input in the form of signal features in a subset of locations in the myocardium. Where the myocardium is represented as a triangular or tetrahedral mesh of points, electrogram information can be provided as a set of features in the mesh points representing the location of measuring electrode leads from an implanted device (such as, for example, an implanted cardiac defibrillator). One example of such features includes the time from pacing to sensed R wave in each of the measurement channels. Electrodes can be used for pacing, such as in non-invasive programmed stimulation, in which case the feature in the pacing lead would be a zero value. Alternative definitions of input features based on measured electrograms could be the polarity of the QRS complex in each lead, upward and downward slopes of the QRS complex in each lead, R wave peak time in each lead, and so forth (it being noted that these features may be available in the data comprising a standard electrocardiogram as well as from an implanted device). The neural network could be trained on a large database of training samples produced by a computational model of cardiac electrophysiology including the presence of an implanted device such as an implanted cardiac defibrillator, capable of delivering pacing stimuli and sensing local electrical activity.

These teachings provide for the automatic determination of ventricular tachycardia exit sites, which can be crucial information for any ventricular tachycardia treatment that locally impacts cardiac tissue (such as, for example, radiofrequency ablation or radiation therapy). Since these teachings only require 12-lead electrocardiogram traces (or fewer or more) and corresponding three-dimensional anatomy information derived from medical imaging approaches, these teachings are fully non-invasive. In particular, these teachings allow the integration of information present in 12-lead electrocardiogram traces print-outs as well as specific electrocardiogram electrode placement derived, for example, from RGB-D camera images, which allows easy integration into existing clinical setups. Also, by integrating the patient's anatomy into the estimation process, these teachings remove considerable uncertainty about the relative orientation of the heart, torso, electrocardiogram electrode orientation. The latter can lead to more accurate estimations.

Further aspects of these teachings are provided by the subject matter of the following clauses (where it will be understood that any of these clauses can be combined with any one of more of the other clauses as desired).

Clause 1. A method to non-invasively identify a ventricular tachycardia exit site in a heart of a particular patient using electrocardiogram signals and medical imagery for the particular patient, the method comprising: by a control circuit: accessing a neural network that has been trained using a plurality of synthetic data examples; inputting information regarding the electrocardiogram signals and the medical imagery for the particular patient to the neural network; outputting from the neural network information that identifies a predicted ventricular tachycardia exit site in the heart of the particular patient.

Clause 2. The method of clause 1 wherein the electrocardiogram signals comprise electrocardiogram signals from no more than twelve electrocardiogram leads.

Clause 3. The method of clause 1 wherein the information regarding the electrocardiogram signals includes information regarding physical placement of electrocardiogram electrodes on the particular patient.

Clause 4. The method of clause 1 wherein the medical imagery comprises at least one of at least one computed tomography image of at least part of the heart and at least one magnetic resonance imaging image of at least part of the heart.

Clause 5. The method of clause 1 wherein the medical imagery comprises three-dimensional imagery of at least part of the heart.

Clause 6. The method of clause 1 wherein the predicted ventricular tachycardia exit site in the heart of the particular patient comprises at least one of: a particular region comprising a predefined subdivision of the heart; a heat map that indicates at least one likelihood of a ventricular tachycardia exit site; and three-dimensional location coordinates.

Clause 7. The method of clause 1 wherein the synthetic data examples include examples that each comprise: information regarding electrocardiogram signals for a representative patient; medical imagery for the representative patient; and at least one labeled ventricular tachycardia exit site.

Clause 8. The method of clause 1 wherein the neural network has also been trained using at least one non-synthetic data example.

Clause 9. The method of clause 1 further comprising: generating at least some of the synthetic data examples using statistical shape modeling to increase variability of at least one of: cardiac and torso anatomy instantiations; medical imagery instantiations; and labeled ventricular tachycardia exit site instantiations.

Clause 10. The method of clause 1 wherein at least some of the synthetic data examples include electrogram information and wherein inputting the information regarding the electrocardiogram signals and the medical imagery for the particular patient to the neural network further includes inputting information regarding electrogram information for the particular patient to the neural network.

Clause 11. An apparatus to non-invasively identify a ventricular tachycardia exit site in a heart of a particular patient using electrocardiogram signals and medical imagery for the particular patient, the apparatus comprising: a control circuit configured to: access a neural network that has been trained using a plurality of synthetic data examples; input information regarding the electrocardiogram signals and the medical imagery for the particular patient to the neural network; and output from the neural network information that identifies a predicted ventricular tachycardia exit site in the heart of the particular patient.

Clause 12. The apparatus of clause 11 wherein the electrocardiogram signals comprise electrocardiogram signals from no more than twelve electrocardiogram leads.

Clause 13. The apparatus of clause 11 wherein the information regarding the electrocardiogram signals includes information regarding physical placement of electrocardiogram electrodes on the particular patient.

Clause 14. The apparatus of clause 11 wherein the medical imagery comprises at least one of at least one computed tomography image of at least part of the heart and at least one magnetic resonance imaging image of at least part of the heart.

Clause 15. The apparatus of clause 11 wherein the medical imagery comprises three-dimensional imagery of at least part of the heart.

Clause 16. The apparatus of clause 11 wherein the predicted ventricular tachycardia exit site in the heart of the particular patient comprises at least one of: a particular region comprising a predefined subdivision of the heart; a heat map that indicates at least one likelihood of a ventricular tachycardia exit site; and three-dimensional location coordinates.

Clause 17. The apparatus of clause 11 wherein the synthetic data examples include examples that each comprise: information regarding electrocardiogram signals for a representative patient; medical imagery for the representative patient; and at least one labeled ventricular tachycardia exit site.

Clause 18. The apparatus of clause 11 wherein the neural network has also been trained using at least one non-synthetic data example.

Clause 19. The apparatus of clause 11 wherein the control circuit is further configured to: generate at least some of the synthetic data examples using statistical shape modeling to increase variability of at least one of: cardiac and torso anatomy instantiations; medical imagery instantiations; and labeled ventricular tachycardia exit site instantiations.

Clause 20. The apparatus of clause 11 wherein at least some of the synthetic data examples include electrogram information and wherein the control circuit is configured to input the information regarding the electrocardiogram signals and the medical imagery for the particular patient to the neural network by further inputting information regarding electrogram information for the particular patient to the neural network.

Clause 21. A non-transitory computer-readable medium for non-invasively identifying a ventricular tachycardia exit site in a heart of a particular patient using electrocardiogram signals and medical imagery for the particular patient, the non-transitory computer-readable medium comprising instructions stored thereon, that when executed on a processor, perform the steps of: accessing a neural network that has been trained using a plurality of synthetic data examples; inputting information regarding the electrocardiogram signals and the medical imagery for the particular patient to the neural network; outputting from the neural network information that identifies a predicted ventricular tachycardia exit site in the heart of the particular patient.

Those skilled in the art will recognize that a wide variety of modifications, alterations, and combinations can be made with respect to the above described embodiments without departing from the scope of the invention, and that such modifications, alterations, and combinations are to be viewed as being within the ambit of the inventive concept.

## Claims

1. A method (200) to non-invasively identify a ventricular tachycardia exit site in a heart of a particular patient (107) using electrocardiogram signals and medical imagery for the particular patient, the method comprising:
by a control circuit (101):
accessing (205) a neural network (104) that has been trained (202) using a plurality of synthetic data examples (203);
inputting (206) information regarding the electrocardiogram signals and the medical imagery for the particular patient to the neural network;
outputting (207) from the neural network information that identifies a predicted ventricular tachycardia exit site in the heart of the particular patient.

2. An apparatus (100) to non-invasively identify a ventricular tachycardia exit site in a heart of a particular patient (107) using electrocardiogram signals and medical imagery for the particular patient, the apparatus comprising:
a control circuit (101) configured to:
access (205) a neural network (104) that has been trained (202) using a plurality of synthetic data examples (203);
input (206) information regarding the electrocardiogram signals and the medical imagery for the particular patient to the neural network; and
output (207) from the neural network information that identifies a predicted ventricular tachycardia exit site in the heart of the particular patient.

3. A non-transitory computer-readable medium (102) for non-invasively identifying a ventricular tachycardia exit site in a heart of a particular patient (107) using electrocardiogram signals and medical imagery for the particular patient, the non-transitory computer-readable medium comprising instructions stored thereon, that when executed on a processor (101), perform the steps of:
accessing (205) a neural network (104) that has been trained (202) using a plurality of synthetic data examples (203);
inputting (206) information regarding the electrocardiogram signals and the medical imagery for the particular patient to the neural network;
outputting (207) from the neural network information that identifies a predicted ventricular tachycardia exit site in the heart of the particular patient.

4. The method, apparatus or computer-readable medium of any of claims 1 through 3 wherein the electrocardiogram signals comprise electrocardiogram signals from no more than twelve electrocardiogram leads (301).

5. The method apparatus or computer-readable medium of any of claims 1 through 4 wherein the information regarding the electrocardiogram signals includes information regarding physical placement of electrocardiogram electrodes on the particular patient.

6. The method apparatus or computer-readable medium of any of claims 1 through 5 wherein the medical imagery comprises at least one of at least one computed tomography image of at least part of the heart and at least one magnetic resonance imaging image of at least part of the heart.

7. The method apparatus or computer-readable medium of any of claims 1 through 6 wherein the medical imagery comprises three-dimensional imagery (304) of at least part of the heart.

8. The method apparatus or computer-readable medium of any of claims 1 through 7 wherein the predicted ventricular tachycardia exit site in the heart of the particular patient comprises at least one of:
a particular region comprising a predefined subdivision of the heart;
a heat map that indicates at least one likelihood of a ventricular tachycardia exit site; and
three-dimensional location coordinates.

9. The method apparatus or computer-readable medium of any of claims 1 through 8 wherein the synthetic data examples include examples that each comprise:
information regarding electrocardiogram signals for a representative patient;
medical imagery for the representative patient; and
at least one labeled ventricular tachycardia exit site.

10. The method apparatus or computer-readable medium of any of claims 1 through 9 wherein the neural network has also been trained (202) using at least one non-synthetic data example (205).

11. The method apparatus or computer-readable medium of any of claims 1 through 10 further comprising:
generating at least some of the synthetic data examples using statistical shape modeling to increase variability of at least one of:
cardiac and torso anatomy instantiations;
medical imagery instantiations; and
labeled ventricular tachycardia exit site instantiations.

12. The method apparatus or computer-readable medium of any of claims 1 through 11 wherein at least some of the synthetic data examples include electrogram information and wherein inputting the information regarding the electrocardiogram signals and the medical imagery for the particular patient to the neural network further includes inputting information regarding electrogram information for the particular patient to the neural network.
